(19) Europäisches Patentamt
European Patent Office
Office européen des brevets

(11) Veröffentlichungsnummer: **0 093 880**
**B1**

(12) # EUROPÄISCHE PATENTSCHRIFT

(45) Veröffentlichungstag der Patentschrift: **06.11.85**

(51) Int. Cl.⁴: **C 07 C 50/04**, C 07 C 46/02, C 07 C 46/08, C 07 C 39/07, C 07 C 37/07

(21) Anmeldenummer: **83103493.9**

(22) Anmeldetag: **11.04.83**

(54) **Verfahren zur Herstellung von 2,3,5- Trimethyl-p-benzochinon.**

(30) Priorität: **23.04.82 DE 3215095**

(43) Veröffentlichungstag der Anmeldung: **16.11.83 Patentblatt 83/46**

(45) Bekanntmachung des Hinweises auf die Patenterteilung: **06.11.85 Patentblatt 85/45**

(84) Benannte Vertragsstaaten: **CH DE FR LI**

(56) *Entgegenhaltungen:*
**EP - A - 0 015 221**
**EP - A - 0 035 635**
**US - A - 3 859 365**

(73) Patentinhaber: **BASF Aktiengesellschaft, Carl-Bosch-Strasse 38, D-6700 Ludwigshafen (DE)**

(72) Erfinder: **Thoemel, Frank, Dr., Leberstrasse 17, D-6940 Weinheim (DE)**
Erfinder: **Hoffmann, Werner, Dr., Ringstrasse 11c, D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft Verfahren zur Herstellung von 2,3,5-Trimethyl-p-benzochinon der Formel I

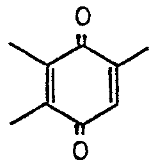

(I)

aus 2,5,6-Trimethyl-2-cyclohexen-1-on der Formel II, 2,3,6-Trimethyl-2-cyclohexen-1-on der Formel III oder 2,3,6-Trimethylphenol der Formel IV jeweils in einem Eintopfprozess.

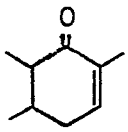

(II)

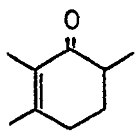

(III)

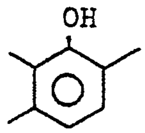

(IV)

Das Trimethyl-p-benzochinon ist unmittelbares Vorprodukt für die Herstellung von Trimethylhydrochinon, das ein essentielles Zwischenprodukt für die Herstellung von Vitamin E ist. Durch den ständig steigenden Bedarf an Vitamin E hat es nicht an Versuchen gefehlt, vorteilhafte Methoden zur Herstellung von Trimethyl-p-benzochinon zu entwickeln.

P.A. Wehrli et al (J. Org. Chem. *37*, 2340 [1972] und DE-OS Nr. 2032081) beschreiben beispielsweise eine indirekte elektrochemische Oxidation des Phenols zum Benzochinon. Hierzu bedarf es allerdings einer speziellen Technologie.

Die Luftoxidation des Trimethylphenols wurde mit gutem Erfolg unter Zuhilfenahme von Kobaltkomplexen vom Typ des «Salcomins» durchgeführt (vgl. DE-OS Nr. 2450908, Schweizer Patentschrift Nr. 501573 und Japanische Auslegeschrift Nr. 7424464). Hierbei ist jedoch der Katalysator recht teuer und muss erst vorgefertigt werden. Zudem werden spezielle Lösungsmittel (Nitrile, Dimethylformamid etc.) verwendet.

Es ist auch bekannt, dass Kupfersalze die Oxidation von alkylierten Phenolen mit Sauerstoff zu den entsprechenden Chinonen katalysieren. Aus der DE-AS Nr. 2221624 geht hervor, dass dazu allerdings unwirtschaftlich grosse Mengen an Kupfersalzen nötig sind.

Die US-Patentschriften Nrn. 3870731 und 3987068 beschreiben die Oxidation mit katalytischen Mengen an Kupfersalzen. Es muss jedoch unter Überdruck gearbeitet werden. In der Regel verwendet man reinen Sauerstoff als Oxidationsmittel, was spezielle Sicherheitsvorkehrungen erfordert.

Wie die angeführten Literaturzitate zeigen, sind die Ausgangsprodukte für die Synthese von alkylsubstituierten Benzochinonen im allgemeinen alkylsubstituierte Phenole. Man gewinnt sie in der Regel durch Dehydrierung der leicht zugänglichen alkylsubstituierten Cyclohexenone in Gegenwart von Platinmetallkatalysatoren (vgl. P.A. Wehrli et al, J. Org. Chem. *37*, 2340 [1972]). Das US-Patent Nr. 3859365 beschreibt die Luftoxidation von alkylsubstituierten Cyclohexenonen. Als Katalysator wird Kupferchlorid in Anwesenheit von wässeriger Salzsäure verwendet.

Aus der Europäischen Patentanmeldung Nr. 0035635 ist weiterhin ein Verfahren zur Herstellung von 2,3,5-Trimethyl-p-benzochinon bekannt, bei dem 2,5,6-Trimethyl-2-cyclohexen-1-on zunächst dehydriert und das erhaltene Produkt dann zum Trimethyl-p-benzochinon oxidiert wird. Dehydrierung und Oxidation erfolgen in demselben organischen Lösungsmittel sowie mit demselben Katalysator. Als Katalysator wird ein Kupfer(II)halogenid verwendet. Nachteilig an diesem Verfahren ist, dass relativ grosse Mengen an Kupfersalzen als Katalysator benötigt werden. Man verwendet hierbei mindestens 1 mol, vorzugsweise 2 bis 5 mol, Kupfersalz pro Mol Ausgangsmaterial.

Es war daher die Aufgabe der Erfindung, das Verfahren zur Herstellung von 2,3,5-Trimethyl-p-benzochinon, bei dem 2,5,6-Trimethyl-2-cyclohexen-1-on zunächst dehydriert und das erhaltene Produkt dann im gleichen Lösungsmittel und mit demselben Katalysator zum Trimethyl-p-benzochinon oxidiert wird, so zu verbessern, dass man mit wesentlich geringeren Katalysatormengen auskommt.

Gegenstand der Erfindung ist ein Verfahren zur Herstellung von Trimethyl-p-benzochinon der Formel I

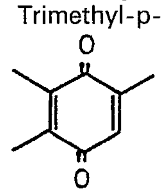

(I)

durch Dehydrieren von 2,5,6-Trimethyl-2-cyclohexen-1-on oder 2,3,6-Trimethyl-2-cyclohexen-1-on in einem inerten organischen Lösungsmittel in Gegenwart von Kupferverbindungen und anschliessende Oxidation des erhaltenen Produkts im gleichen inerten organischen Lösungsmittel in Gegenwart einer Kupferverbindung, das dadurch gekennzeichnet ist, dass man

A. das 2,5,6-Trimethyl-2-cyclohexen-1-on oder 2,3,6-Trimethyl-2-cyclohexen-1-on in Gegenwart von weniger als molaren Mengen von Kupfer-I- oder -II-oxid sowie in Gegenwart von 2 bis 10 mol eines Halogenwasserstoffgases pro Mol Trimethylcyclohexenon in einem praktisch wasserfreien niedermolekularen Alkanol bei Reaktionstemperaturen von 0 bis 150° C, vorzugsweise 20 bis 120° C mit Luft oder Sauerstoff umsetzt, dann

B. das im wesentlichen 2,3,6-Trimethyl-phenol und/oder 4-Halogen-2,3,6-trimethyl-phenol enthaltende Reaktionsgemisch mit soviel eines Alkalialkoholats versetzt, dass eine Probe nach Anfeuchten mit Wasser einen pH-Wert von 3 bis 6, vorzugsweise 4,0 bis 4,4, anzeigt und anschliessend bei Temperaturen von 0 bis 150° C, vorzugsweise bei 20 bis 120° C mit Luft, reinem Sauerstoff oder tert.-Butylhydroperoxid umsetzt.

Gegenstand der Erfindung ist weiterhin ein Verfahren zur Herstellung von Trimethyl-p-benzochi-

non der Formel I, das dadurch gekennzeichnet ist, dass man in ein Gemisch aus praktisch wasserfreiem niedermolekularem Alkanol und weniger als molaren Mengen Kupfer-I- oder -II-oxid bei 0 bis 30° C 1 bis 10 Moläquivalent eines Halogenwasserstoffgases, pro Mol umzusetzendes Substrat, einleitet und danach mit soviel Alkalialkoholat versetzt, dass eine Probe nach Anfeuchten mit Wasser einen pH-Wert von 3 bis 6, vorzugsweise 4,0 bis 4,4, insbesondere etwa 4,2, anzeigt, danach etwa ein Moläquivalent 2,3,6-Trimethylphenol zusetzt und das Reaktionsgemisch anschliessend mit Luft, Sauerstoff oder tert.-Butylhydroperoxid bei Reaktionstemperaturen von 0 bis 150° C, vorzugsweise bei 20 bis 120° C, umsetzt.

Es war überraschend, dass es gelang, Reaktionsbedingungen zu finden, unter denen man 2,5,6-Trimethyl-2-cyclohexen-1-on bzw. 2,3,6-Trimethyl-2-cyclohexen-1-on in einer Eintopfreaktion in das begehrte 2,5,6-Trimethyl-p-benzochinon überführen kann, auch ohne molare oder mehr als molare Mengen der Kupferverbindungen einzusetzen, ohne die Notwendigkeit unbedingt reinen Sauerstoff für Dehydrierung und/oder Oxidation verwenden zu müssen und ohne mit höheren Luft- oder Sauerstoffdrücken arbeiten zu müssen.

Bei der erfindungsgemässen Umsetzung wird in eine Lösung von 2,5,6-Trimethyl-2-cyclohexen-1-on in niederen Alkanolen in Gegenwart von katalytischen Mengen von Kupfer-(I)- oder Kupfer-(II)-verbindungen und gleichzeitiger Anwesenheit von Halogenwasserstoff Luft oder Sauerstoff eingeleitet. Durch gaschromatographische Analyse (GCA) lässt sich feststellen, dass bei der daraufhin einsetzenden Reaktion zunächst 2,3,6-Trimethyl-2-cyclohex-1-on und dann 2,3,6-Trimethylphenol gebildet wird. Mit zunehmender Reaktionszeit wandelt sich das 2,3,6-Trimethylcyclohexen-1-on vollständig in 2,3,6-Trimethylphenol um. Gibt man nun zum Reaktionsgemisch so viel Alkalialkoholat, dass eine Probe nach Anfeuchten mit Wasser einen pH-Wert von 3 bis 6, vorzugsweise etwa 4,2, ergibt und leitet weiter Luft oder Sauerstoff ein, so wird überraschend das Phenol in guten Ausbeuten in 2,3,5-Trimethyl-p-benzochinon umgewandelt. Diese zuletzt genannte Umwandlung ist insofern als überraschend zu bezeichnen, als sich 2,3,6-Trimethylphenol in nahezu neutralen, oder alkalischen alkoholischen Lösungen in Gegenwart katalytischer Mengen von Kupferverbindungen nicht unter Normaldruck durch Luft oder Sauerstoff in 2,3,5-Trimethylbenzochinon umwandeln lässt.

Wenn man das Reaktionsgemisch aus 2,5,6-Trimethyl-2-cyclohexen-1-on, Alkanol, katalytischen Mengen an Kupferoxid und Halogenwasserstoffgas wie oben beschrieben, länger mit Luft bzw. Sauerstoff reagieren lässt, so erhält man neben 2,3,6-Trimethyl-phenol 4-Halogen-2,3,6-Trimethylphenol. Wird dieses Produkt nicht isoliert sondern das Reaktionsprodukt wie oben beschrieben mit Alkalialkoholat behandelt und weiter mit Luft bzw. Sauerstoff oxidiert, so erhält man hieraus ebenfalls 2,3,5-Trimethyl-p-benzochinon.

Die Oxidation der nach Reaktionsschritt A hergestellten Reaktionsprodukte kann nach der Behandlung mit Alkalialkoholat anstatt mit Luft oder Sauerstoff auch durch tert.-Butylhydroperoxid erfolgen. Je nachdem, ob man überwiegend Trimethylphenol oder Trimethyl-halogenphenol hergestellt hat, erhält man bei der nachfolgenden Oxidation mit tert.-Butylhydroperoxid das 2,3,5-Trimethyl-p-benzochinon mit Ausbeuten zwischen 65 und 80% d.Th.

Mit Hilfe des neuen Katalysatorsystems gelingt es auch, 2,3,6-Trimethyl-phenol in das 2,3,5-Trimethyl-p-benzochinon zu überführen. Zu diesem Zweck muss das Katalysatorsystem durch Umsetzung von niederem Alkanol, Halogenwasserstoff, Kupfer-I-oxid und Luft und durch nachfolgende Reaktion mit Alkalialkoholat in der oben beschriebenen Weise vorgefertigt werden.

Das erfindungsgemässe Verfahren ist auch technisch auf einfache Weise durchführbar.

Als Lösungsmittel werden erfindungsgemäss nur niedermolekulare und praktisch wasserfreie Alkanole, wie Methanol, Ethanol, Propanol, Isopropanol oder tert.-Butanol verwendet. Man braucht nur soviel Lösungsmittel einzusetzen, wie zur Auflösung des zu oxidierenden Substrats nötig ist. Allerdings muss dafür gesorgt werden, dass dem Reaktionsgemisch keine nennenswerten Mengen an Wasser zugefügt werden, da die Oxidation zum Chinon bei Anwesenheit von Wasser misslingt.

Die erfindungsgemässen Reaktionen werden im allgemeinen bei Normaldruck und bei einer Temperatur unterhalb der Siedetemperatur bis zur Siedetemperatur der eingesetzten Lösungsmittel durchgeführt. Mit Vorteil arbeitet man zwischen 20 und 100° C, insbesondere 40 bis 80° C. Die Umsetzung wäre prinzipiell auch in einem Druckgefäss unter Aufpressen von Luft oder Sauerstoff durchführbar, jedoch bringt das Arbeiten unter Druck keine wesentlichen Vorteile gegenüber dem Arbeiten unter Normaldruck mit sich. In der Regel wird daher die Luft oder der Sauerstoff in das Reaktionsgemisch eingeleitet. Die zur Oxidation für ein Mol Substrat erforderliche Luftmenge liegt zwischen 10 bis 2000 Liter/Stunde, vorzugsweise zwischen 40 und 1000 Liter/Stunde.

Bei der Oxidation der 2,3,6-Trimethylphenol oder 4-Halogen-2,3,6-trimethyl-phenol enthaltenden Reaktionsgemische mit tert.-Butylhydroperoxid setzt man das Peroxid in möglichst äquimolaren Mengen ein. Man kann aber auch die durch Luft oder Sauerstoff bewirkte Oxidation zum Benzochinon durch Zusätze von tert.-Butylhydroperoxid unterstützen. Entsprechend den jeweiligen Reaktionsbedingungen wird man auch mit weniger als einem Mol tert.-Butylhydroperoxid, bezogen auf das zu oxidierende Substrat, auskommen. Das tert.-Butylhydroperoxid wird zweckmäsig bei Temperaturen zwischen 35 und 100° C im Verlauf von 1 bis 4 Stunden zum gerührten Reaktionsgemisch gegeben. Anschliessend lässt man noch ca. zwei Stunden nachreagieren.

Die Reaktionsdauer kann je nach den gewählten

Reaktionsbedingungen zwischen wenigen Stunden und einigen Tagen betragen.

Der vor der Oxidation der Phenole zum Benzochinon erfindungsgemäss nötige Zusatz von Alkalialkoholat muss möglichst genau dosiert werden. Man verwendet zweckmässig Natriummethylat oder Natriummethylat in Lösung von niederen Alkanolen wie Methanol oder Ethanol. Unter Rühren wird soviel von der alkoholischen Alkalialkoholatlösung zum Reaktionsgemisch zugesetzt, dass eine Probe nach Anfeuchten mit Wasser einen pH-Wert zwischen 3 bis 6, vorzugsweise 4,0 bis 4,4, insbesondere etwa 4,2, ergibt.

Das bei der erfindungsgemässen Umsetzung erhaltene Reaktionsgemisch wird im allgemeinen folgendermassen aufgearbitet. Man engt es durch Abdestillieren des Lösungsmittels auf etwa 1/3 seines Volumens ein, gibt etwa dasselbe Volumen an Wasser zu, extrahiert mit der gleichen Menge an Toluol, wäscht mit Wasser neutral, falls der pH-Wert nicht zwischen 5,5 und 7 liegt, und destilliert.

Trimethyl-p-benzochinon ist ein wichtiges Zwischenprodukt für die Herstellung von Vitamin E.

*Beispiel 1*

55 g Chlorwasserstoff wurden bei Raumtemperatur in 400 ml Isopropanol eingeleitet und in die erhaltene Lösung 28 g (0,2 mol) 2,5,6-Trimethyl-2-cyclohexen-1-on sowie 4 g (0,028 mol) Kupfer-I-oxid gegeben. Anschliessend wurde das Reaktionsgemisch auf 70° C erhitzt und 3 Stunden lang ein Luftstrom von 75 Litern/Stunde unter Rühren eingeleitet. Laut gaschromatographischer Analyse (2 m OV 17, 200° C) war dann kein 2,5,6-Trimethyl-2-cyclohexen-1-on mehr nachweisbar, sondern 2,3,6-Trimethylphenol neben wenig 4-Chlor-2,3,6-trimethylphenol entstanden. Dann liess man bei Raumtemperatur 234 ml einer 30%igen wässerigen Natriummethylatlösung in Methanol zulaufen. Eine Probe des Reaktionsgemisches zeigte nach Versetzen mit Wasser einen pH-Wert von 4,2 an. Danach wurde das Gemisch auf 62° C erhitzt und der Luftstrom von 75 Litern/Stunde, der während des gesamten Prozesses nicht unterbrochen wurde, für weitere 16 Stunden in das auf 62° C erwärmte Gemisch eingeleitet.

Zur Aufarbeitung destillierte man das Lösungsmittel weitgehend ab und goss den Rückstand auf etwa das gleiche Volumen Wasser. Nach Extraktion mit 200 ml Toluol, Waschen des Extraktes mit Wasser und Einengen der organischen Phase am Rotationsverdampfer destillierte man den Rückstand über eine Claisenbrücke. Bei 61 bis 115° C bei 0,1 mbar gingen 21,9 g eines Produktes über, welches laut gaschromatographischer Analyse (GCA) 85% 2,3,5-Trimethyl-p-benzochinon und 10% 4-Chlor-2,3,6-trimethyl-phenol enthielt. Die Ausbeute an 2,3,5-Trimethyl-p-benzochinon betrug somit 62% d.Th.

*Beispiel 2*

45 g Chlorwasserstoff wurden bei Raumtemperatur in 400 ml Isopropanol eingeleitet und diese Lösung mit 4 g (0,028 mol) $Cu_2O$ und 28 g (0,2 mol) 2,5,6-Trimethyl-2-cyclohexen-1-on versetzt, das Ganze auf 70° C erhitzt und für 2 Stunden unter Rühren bei 70° C ein Luftstrom von 75 l/h eingeleitet. Dann war gemäss GCA alles Trimethylcyclohexenon in Trimethylphenol umgewandelt. Anschliessend wurde das Reaktionsgemisch mit 180 ml einer 30%igen $NaOCH_3$-Lösung in $CH_3OH$ versetzt (pH nach Versetzen der Probe war dann 4,2), dann auf 40° C erhitzt und im Verlauf von 2 Stunden unter Rühren mit 28 g (0,3 mol) tert.-Butylhydroperoxid versetzt und schliesslich noch 2 Stunden bei 40° C nachgerührt.

Zur Aufarbeitung destillierte man das Lösungsmittel weitgehend ab, goss den Rückstand auf das gleiche Volumen an Wasser und extrahierte mit 900 ml Toluol. Nach Waschen mit Wasser und Einengen der organischen Phase am Rotationsverdampfer destillierte man den Rückstand an einer Claisenbrücke. Man erhielt 21,4 g eines Produktes, welches gemäss GCA zu 89,5% aus 2,3,5-Trimethyl-p-benzochinon besteht. Die Ausbeute beträgt somit 64% d.Th.

*Beispiel 3*

52 g HCl-Gas wurden bei Raumtemperatur in 400 ml Isopropanol eingeleitet und die erhaltene Lösung mit 4 g (0,028 mol) $Cu_2O$ und 28 g (0,2 mol) 2,5,6-Trimethyl-2-cyclohexen-1-on versetzt, das Ganze auf 70° C erhitzt und für 14 Stunden unter Rühren ein Luftstrom von 75 l/h eingeleitet. Gemäss GCA lagen im Reaktionsgemisch dann 94% 4-Chlor-2,3,6-trimethyl-phenol neben 4% 2,3,6-Trimethyl-phenol vor. Anschliessend wurde das Reaktionsgemisch bei 10 bis 20° C mit 150 ml einer 30%igen $NaOCH_3$-Lösung in $CH_3OH$ versetzt (pH der Probe nach Versetzen mit Wasser = 4,2), auf 40° C erwärmt, im Verlauf von 2 Stunden unter Rühren bei 40° C mit 19 g (0,2 mol) tert.-Butylhydroperoxid versetzt und noch 7 Stunden bei 40° C gerührt. Laut GCA waren dann 75% des gebildeten 4-Chlor-trimethylphenols umgesetzt. Anschliessend wurden innerhalb von 1 Stunde bei 40° C nochmals 10 g (0,1 mol) tert.-Butylhydroperoxid zugefügt und das Reaktionsgemisch noch 3 Stunden bei 40° C nachgerührt. Aufarbeitung wie in Beispiel 1 beschrieben ergab 25,2 g eines Produktes, welches gemäss GCA zu 95% aus 2,3,5-Trimethyl-p-benzochinon besteht. Die Ausbeute betrug somit 80% d.Th.

*Beispiel 4*

53 g HCl-Gas wurden bei Raumtemperatur in 400 ml Isopropanol eingeleitet und diese Lösung mit 4 g (0,028 mol) $Cu_2O$ und nach 5minutigem Rühren noch bei 10 bis 20° C 300 ml einer 30%igen $NaOCH_3$-Lösung in $CH_3OH$ versetzt (pH der Probe nach Versetzen mit Wasser = 4,2). Zu dem erhaltenen Reaktionsgemisch addierte man anschliessend rasch 28 g (0,2 mol) 2,3,6-Trimethyl-phenol, erhitzte es auf 60° C und leitete 21 Stunden einen Luftstrom von 75 l/h ein. Gemäss CGA war dann das Trimethylphenol vollständig in 2,3,6-Trimethyl-p-benzochinon umgewandelt.

Bei einer Aufarbeitung analog Beispiel 1 erhielt

man 21,74 g eines Produktes, welches zu 99,5% aus 2,3,5-Trimethyl-p-benzochinon bestand. Die Ausbeute betrug somit 72% d.Th.

*Beispiel 5*

10 g HCl-Gas wurden bei Raumtemperatur in 400 ml Isopropanol eingeleitet und die Lösung mit 4 g (0,028 mol) $Cu_2O$ versetzt, auf 70° C erhitzt und 4 Stunden lang ein Luftstrom von 75 l/h eingeleitet. Anschliessend addierte man bei Raumtemperatur 24 ml einer 30%igen $NaOCH_3$-Lösung in $CH_3OH$ (pH der Probe nach Versetzen mit Wasser = 4,2), sowie 28 g (0,2 mol) 2,3,6-Trimethylphenol und nach Erhitzen auf 40° C im Verlauf von 2 Stunden 18 g (0,2 mol) tert.-Butylhydroperoxid. Gleichzeitig leitete man einen Luftstrom von 75 l/h in das Reaktionsgemisch ein. Nach einer Reaktionszeit von 12 Stunden arbeitete man wie in Beispiel 1 beschrieben auf. Man erhielt 22,54 g eines 99,7% reinen 2,3,5-Trimethyl-p-benzochinons. Die Ausbeute betrug somit 76% d.Th.

## Patentansprüche

1. Verfahren zur Herstellung von Trimethyl-p-benzochinon der Formel I

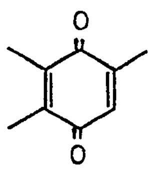

(I)

durch Dehydrieren von 2,5,6-Trimethyl-2-cyclohexen-1-on oder 2,3,6-Trimethyl-2-cyclohexen-1-on in einem inerten organischen Löungsmittel in Gegenwart von Kupferverbindungen und anschliessende Oxidation des erhaltenen Produkts im gleichen inerten organischen Lösungsmittel in Gegenwart einer Kupferverbindung, dadurch gekennzeichnet, dass man

A. das 2,5,6-Trimethyl-2-cyclohexen-1-on oder 2,3,6-Trimethyl-2-cyclohexen-1-on in Gegenwart von weniger als molaren Mengen von Kupfer-I- oder -II-oxid sowie in Gegenwart von 1 bis 10 mol eines Halogenwasserstoffgases pro Mol Trimethylcyclohexenon in einem praktisch wasserfreien niedermolekularen Alkanol bei Reaktionstemperaturen von 0 bis 150° C mit Luft oder Sauerstoff umsetzt, dann

B. das im wesentlichen 2,3,6-Trimethyl-phenol und/oder 4-Halogen-2,3,6-trimethyl-phenol enthaltende Reaktionsgemisch mit soviel eines Alkalialkoholats versetzt, dass eine Probe nach Anfeuchten mit Wasser einen pH-Wert von 3 bis 6 anzeigt und anschliessend bei Temperaturen von 0 bis 150° C mit Luft, reinem Sauerstoff oder tert.-Butylhydroperoxid umsetzt.

2. Verfahren gemäss Anspruch 1, dadurch gekennzeichnet, dass man im Reaktionsschritt B das Reaktionsgemisch mit soviel eines Alkalialkoholats versetzt, dass eine Probe nach Anfeuchten mit Wasser einen pH-Wert von 4,0 bis 4,4 anzeigt.

3. Verfahren zur Herstellung von Trimethyl-p-benzochinon der Formel I, dadurch gekennzeichnet, dass man in ein Gemisch auspraktisch wasserfreiem niedermolekularem Alkanol und weniger als molaren Mengen Kupfer-I- oder -II-oxid bei 0 bis 30° C 1 bis 10 Moläquivalent eines Halogenwasserstoffgases pro Mol umzusetzendes Substrat einleitet und danach mit soviel Alkalialkoholat versetzt, dass eine Probe nach Anfeuchten mit Wasser einen pH-Wert von 3 bis 6 anzeigt, danach etwa ein Moläquivalent 2,3,6-Trimethyl-phenol zusetzt und das Reaktionsgemisch anschliessend mit Luft, Sauerstoff oder tert.-Butylhydroperoxid bei Reaktionstemperaturen von 0 bis 150° C umsetzt.

## Claims

1. A process for the preparation of trimethyl-p-benzoquinone of the formula I

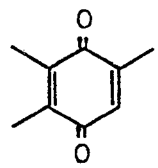

(I)

by dehydrogenation of 2,5,6-trimethylcyclohex-2-en-1-one or 2,3,6-trimethylcyclohex-2-en-1-one in an inert organic solvent in the presence of a copper compound, followed by oxidation of the resulting product in the same inert organic solvent in the presence of a copper compound, wherein

A. 2,5,6-trimethylcyclohex-2-en-1-one or 2,3,6-trimethylcyclohex-2-en-1-one is reacted with air or oxygen at from 0 to 150°C in a virtually anhydrous low molecular weight alkanol in the presence of a less than molar amount of copper(I) oxide or copper(II) oxide and in the presence of from 1 to 10 moles of a hydrogen halide gas per mole of trimethylcyclohexenone, and thereafter

B. the reaction mixture, which essentially contains 2,3,6-trimethylphenol and/or 4-halo-2,3,6-trimethylphenol, has an alkali metal alcoholate added to it in such an amount that a sample, when moistened with water, has a pH of from 3 to 6, and is then reacted with air, pure oxygen or tert.-butyl hydroperoxide at from 0 to 150° C.

2. A process as claimed in Claim 1, wherein, in step B, the reaction mixture has an alkali metal alcoholate added to it in such an amount that a sample, when moistened with water, has a pH of from 4.0 to 4.4.

3. A process for the preparation of trimethyl-p-benzoquinone of the formula I, wherein from 1 to 10 mole equivalents of a hydrogen halide gas per mole of substrate to be reacted are passed into a mixture of a virtually anhydrous low molecular weight alkanol and a less than molar amount of copper(I) oxide or copper(II) oxide at from 0 to 30° C, an alkali metal alcoholate is added in such an amount that a sample, when moistened with water, has a pH of from 3 to 6, about one mole equivalent of 2,3,6-trimethylphenol is added and the mixture is then reacted with air, oxygen or tert.-butyl hydroperoxide at from 0 to 150° C.

## Revendications

1. Procédé pour la préparation de triméthyl-p-benzoquinone de formule I

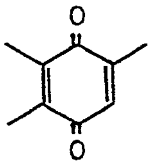

(I)

par déshydrogénation de 2,5,6-triméthyl-2-cyclohexène-1-one ou de 2,3,6-triméthyl-2-cyclohexène-1-one dans un solvant organique inerte en présence de composés du cuivre, suivie d'oxydation du produit obtenu dans le même solvant organique inerte en présence d'un composé du cuivre, caractérisé en ce que

A. l'on fait réagir avec l'air ou l'oxygène la 2,5,6-triméthyl-2-cyclohexène-1-one ou la 2,3,6-triméthyl-2-cyclohexène-1-one en présence de quantités d'oxyde cuivreux ou cuivrique inférieures aux quantités molaires, ainsi qu'en présence de 1 à 10 mol d'un gaz halohydrique par mole de triméthylcyclohexénone dans un alcanol inférieur pratiquement anhydre, à des températures de réaction de 0 à 150° C, puis

B. l'on additionne le mélange réactionnel, qui contient essentiellement du 2,3,6-triméthylphénol et/ou un 4-halogéno-2,3,6-triméthylphénol, d'une quantité suffisante d'un alcoolate alcalin pour qu'un échantillon présente, après humidification à l'eau, un pH de 3 à 6, puis on le fait réagir, à des températures de 0 à 150° C, avec de l'air, de l'oxygène pur ou de l'hydroperoxyde de butyle tertiaire.

2. Procédé selon la revendication 1, caractérisé en ce que, dans la phase de réaction B, on additionne le mélange réactionnel d'une quantité suffisante d'un alcoolate alcalin pour qu'un échantillon présente, après humidification à l'eau, un pH de 4,0 à 4,4.

3. Procédé pour la préparation de triméthyl-p-benzoquinone de formule I, caractérisé en ce qu'on fait passer, à 0-30° C, dans un mélange d'alcanol inférieur pratiquement anhydre et de quantités d'oxyde cuivreux ou cuivrique inférieures à la quantité molaire, 1 à 10 équivalents molaires d'un gaz halohydrique par mole de substrat à faire réagir, puis on ajoute suffisamment d'alcoolate alcalin pour qu'un échantillon présente, après humidification à l'eau, un pH de 3 à 6, après quoi on ajoute à peu près un équivalent molaire de 2,3,6-triméthylphénol et on fait ensuite réagir le mélange réactionnel avec de l'air, de l'oxygène ou de l'hydroperoxyde de butyle tertiaire à une température de réaction de 0 à 150° C.